(19) Europäisches Patentamt
European Patent Office
Office européen des brevets

(11) Veröffentlichungsnummer: **0 337 151 A2**

(12) # EUROPÄISCHE PATENTANMELDUNG

(21) Anmeldenummer: 89104758.1

(22) Anmeldetag: 17.03.89

(51) Int. Cl.[4]: C07D 401/12 , C07D 413/12 , C07D 487/04 , C07D 405/12 , C07D 409/12 , C07D 403/12 , C07D 521/00 , C07D 417/12 , C07D 471/04 , C07D 213/30 , A01N 43/38

(30) Priorität: 30.03.88 JP 74639/88

(43) Veröffentlichungstag der Anmeldung: 18.10.89 Patentblatt 89/42

(84) Benannte Vertragsstaaten: BE CH DE FR GB IT LI NL

(71) Anmelder: NIHON TOKUSHU NOYAKU SEIZO K.K.
Itohpia Nihonbashi·Honcho Building 7-1, Nihonbashi Honcho 2-chome
Chuo-ku Tokyo 103(JP)

(72) Erfinder: Kume, Toyohiko
6-7-8 Asahigaoka
Hino-shi Tokyo(JP)
Erfinder: Goto, Toshio
3454-21, Honmachida
Machida-shi Tokyo(JP)
Erfinder: Kamochi, Atsumi
2-24-10, Higashi-Toyoda
Hino-shi Tokyo(JP)
Erfinder: Shibuya, Katsuhiko
39-15, Namiki-cho
Hachioji-shi Tokyo(JP)
Erfinder: Miyauchi, Hiroshi
39-15, Namiki-cho
Hachioji-shi Tokyo(JP)

(74) Vertreter: Schumacher, Günter, Dr. et al
c/o Bayer AG Konzernverwaltung RP Patentabteilung
D-5090 Leverkusen 1 Bayerwerk(DE)

(54) **N-Substituierte phenyl-heterocyclische Verbindungen.**

(57) Die vorliegende Erfindung betrifft N-substituierte phenyl-heterocyclische Verbindungen der Formel (I)

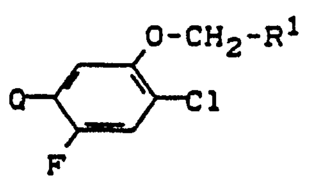

(I)

in der
Q die folgenden Gruppen

EP 0 337 151 A2

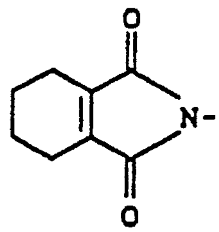 oder 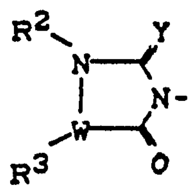

bezeichnet und

R' eine gegebenenfalls substituierte 5- oder 6-gliedrige heterocyclische Gruppe bezeichnet, sowie Verfahren und neue Zwischenprodukte zu ihrer Herstellung und ihre Verwendung als Herbizide.

## N-Substituierte phenyl-heterocyclische Verbindungen

Die vorliegende Erfindung betrifft neue N-substituierte phenyl-heterocyclische Verbindungen, Verfahren und neue Zwischenprodukte zu ihrer Herstellung und ihre Verwendung als Herbizide.

Es wurde bereits offenbart, daß bestimmte Tetrahydrophthalimide herbizide Aktivitäten aufweisen (siehe die US-PS 4 332 944).

Nunmehr wurden neue N-substituierte phenyl-heterocyclische Verbindungen der Formel (I)

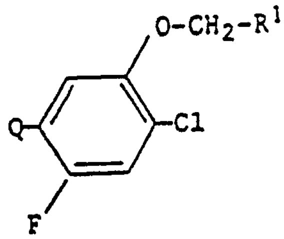

(I)

gefunden, in der
Q die folgenden Gruppen

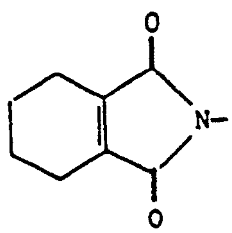

oder

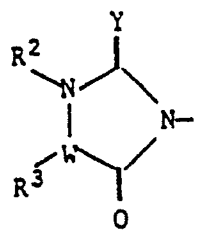

,

bezeichnet, worin
$R^2$ zusammen mit $R^3$ Tetramethylen oder die nachstehende Gruppe bildet

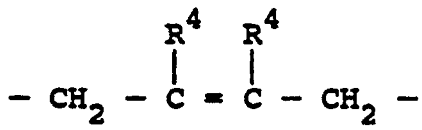

,

$R^4$ Wasserstoff oder Methyl bezeichnet,
Y O oder S bezeichnet,
W - $\overset{|}{C}$H- oder - $\overset{|}{N}$ - bezeichnet und
$R^1$ eine gegebenenfalls substituierte 5- oder 6-gliedrige heterocyclische Gruppe bezeichnet.

Die Verbindungen der Formel (I) werden mittels eines Verfahrens erhalten, bei dem

a) in dem Fall, in dem Q die folgende Gruppe

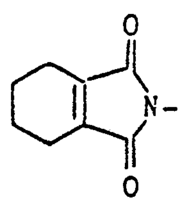

ist, Verbindungen der Formel (II)

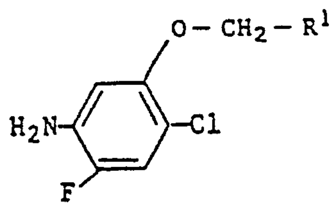

(II)

,

in denen $R^1$ die im Vorstehenden angegebenen Bedeutungen hat, mit 3,4,5,6-Tetrahydrophthalsäureanhydrid in Gegenwart inerter Lösungsmittel umgesetzt werden

oder

b) in dem Fall, in dem Q die folgende Gruppe

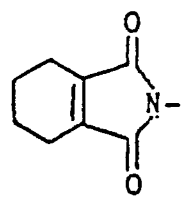

ist, 2-Chloro-4-fluoro-5-(4,5,6,7-tetrahydro-2H-isoindol-1,3-dion-2-yl)phenol mit Verbindungen der Formel (III)

$M-CH_2-R^1$ (III),

in denen

$R^1$ die im Vorstehenden angegebenen Bedeutungen hat und

M Halogen, $C_1$-$C_4$-Alkylsulfonyloxy, Phenylsulfonyloxy oder p-Toluolsulfonyloxy bezeichnet,

oder einem Salz der Verbindungen der Formel (III) in Gegenwart eines inerten Lösungsmittels und gegebenenfalls in Gegenwart einer Base umgesetzt werden

oder

c) in dem Fall, in dem Q die folgende Gruppe

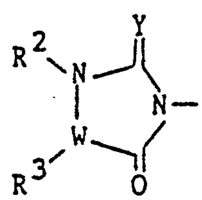

ist, Verbindungen der Formel (IV)

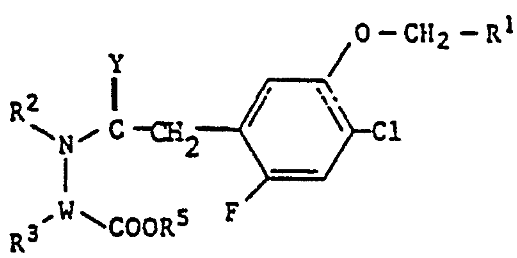

(IV)

,

in denen

$R^2$, $R^3$, Y, W und $R^1$ die im Vorstehenden angegebenen Bedeutungen haben und

$R^5$ Wasserstoff oder Niederalkyl bezeichnet,

einer Ringschluß-Kondensationsreaktion in Gegenwart eines basischen Katalysators und in Gegenwart eines inerten Lösungsmittels unterworfen werden.

Die N-substituierten phenyl-heterocyclischen Verbindungen der Formel (I) gemäß der vorliegenden Erfindung zeigen eine starke herbizide Aktivität.

Überraschenderweise zeigen die erfindungsgemäßen N-substituierten phenyl-heterocyclischen Verbindungen eine wesentlich stärkere herbizide Wirkung als diejenigen, die aus dem Stand der Technik, beispielsweise aus der oben genannten US-PS, bekannt sind.

Unter den erfindungsgemäßen N-substituierten phenyl-heterocyclischen Verbindungen der Formel (I) sind bevorzugte Verbindungen diejenigen, in denen
Q die folgenden Gruppen

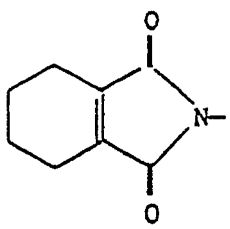

oder

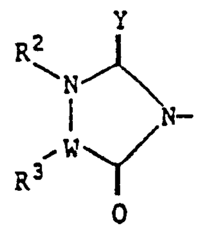

bezeichnet, worin
$R^2$ zusammen mit $R^3$ Tetramethylen oder die nachstehende Gruppe bildet
$-CH_2CH=CHCH_2-$ ,
Y O oder S bezeichnet,
W -CH- oder -N- bezeichnet und
$R^1$ eine gegebenenfalls substituierte 5-gliedrige heterocyclische oder 6-gliedrige aromatisch-heterocyclische Gruppe mit wenigstens einem Stickstoff-Atom-bezeichnet.

Ganz besonders bevorzugte N-substituierte phenylheterocyclische Verbindungen der Formel (I) sind diejenigen, in denen
Q die folgenden Gruppen

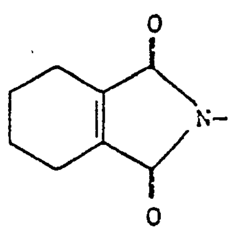

oder

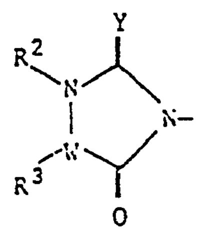

bezeichnet, worin
$R^2$ zusammen mit $R^3$ Tetramethylen oder die nachstehende Gruppe bildet
$-CH_2CH=CHCH_2-$ ,
Y O oder S bezeichnet,
W -CH- oder -N- bezeichnet und
$R^1$ eine gegebenenfalls substituierte 5-gliedrige heterocyclische Gruppe mit wenigstens einem Stickstoff-Atom und wenigstens einem Sauerstoff- oder Schwefel-Atom oder eine gegebenenfalls substituierte 6-gliedrige aromatisch-heterocyclische Gruppe mit 1 oder 2 Stickstoff-Atomen bezeichnet.

Im allgemeinen bezeichnet $R^1$ in der Formel (I) und in den anderen Formeln, in denen es vorkommt, eine Gruppe, die ausgewählt ist aus der aus 5-gliedrigen heterocyclischen Gruppen, z.B. Tetrahydrofuran, Furan, Thiophen, Pyrazol, Imidazol, 1,2,4-Triazol, Tetrazol, Isoxazol, Oxazol, 1,2,4-Oxadiazol, 1,2,5-Oxadiazol, 1,3,4-Oxadiazol, Isothiazol, Thiazol, 1,2,3-Thiadiazol, 1,2,4-Thiadiazol, 1,2,5-Thiadiazol, 1,3,4-Thiadiazol und dergleichen, und aus 6-gliedrigen aromatischen heterocyclischen Gruppen, z. B. Pyridin, Pyrimidin, Pyrazin, 1,3,5-Triazin und dergleichen bestehenden Klasse. Die Ringe in diesen Gruppen können einen oder mehrere, vorzugsweise einen oder zwei für 5-Ringe, oder einen, zwei oder drei für 6-Ringe, gleiche oder verschiedene Substituenten haben, die aus der aus Amino, Methylamino, Ethylamino, Dimethylamino, Formylamino, Acetylamino, Fluoro, Chloro, Bromo, Methoxy, Ethoxy, Methylthio, Ethylthio, Methyl, Ethyl,

Isopropyl, Trichloromethyl, Trifluoromethyl und Methoxymethyl bestehenden Klasse ausgewählt sind.

Vorzugsweise bezeichnet $R^1$ Pyrazol, Imidazol, 1,2,4-Triazol, Isoxazol, 1,2,4-Oxadiazol, 1,2,5-Oxadiazol, 1,3,4-Oxadiazol, Thiazol, 1,2,3-Thiadiazol, 1,2,4-Thiadiazol, 1,2,5-Thiadiazol, 1,3,4-Thiadiazol, Pyridin oder Pyrazin. Die Ringe in diesen Gruppen können einen oder zwei für 5-Ringe, oder einen, zwei oder drei für 6-Ringe, gleiche oder verschiedene Substituenten haben, die aus der aus Methyl, Ethyl, Methoxy und Ethoxy bestehenden Klasse ausgewählt sind.

Beispiele für Verbindungen der Formel (I) gemäß der vorliegenden Erfindung sind die folgenden:

2-[4-Chloro-2-fluoro-5-(pyridin-2-yl-methoxy)phenyl]-4,5,6,7-tetrahydro-2H-isoindol-1,3-dion;

2-[4-Chloro-2-fluoro-5-(isoxazol-3-yl-methoxy)phenyl]-4,5,6,7-tetrahydro-2H-isoindol-1,3-dion;

4-[4-Chloro-2-fluoro-5-(pyridin-2-yl-methoxy)phenyl]-1,2-tetramethylen-1,2,4-triazolidin-3,5-dion; und

2-[4-Chloro-2-fluoro-5-(4-methyl-1,2,5-oxadiazol-3-yl-methoxy)phenyl]-4,5,6,7-tetrahydro-2H-isoindol-1,3-dion.

Wenn als Ausgangsstoffe in dem Verfahren (a) beispielsweise 4-Chloro-2-fluoro-5-(pyridin-2-ylmethoxy)-anilin und 3,4,5,6-Tetrahydrophthalsäureanhydrid eingesetzt werden, kann die Reaktion durch das folgende Schema dargestellt werden:

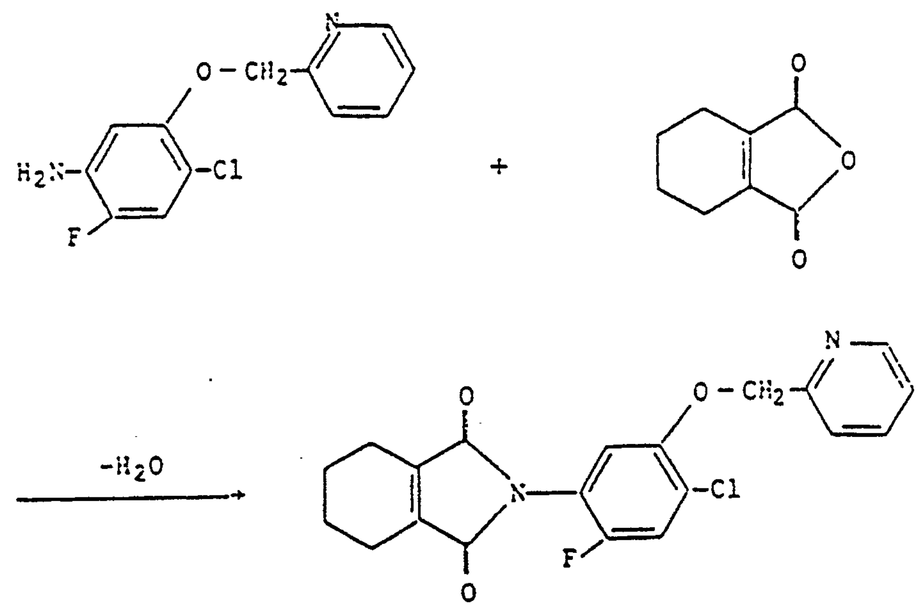


Wenn als Ausgangsstoffe in dem Verfahren (b) beispielsweise 2-Chloro-4-fluoro-5-(4,5,6,7-tetrahydro-2H-isoindol-1,3-dion-2-yl)phenol und 3-(Chloromethyl)isoxazol eingesetzt werden, kann die Reaktion durch das folgende Schema dargestellt werden:

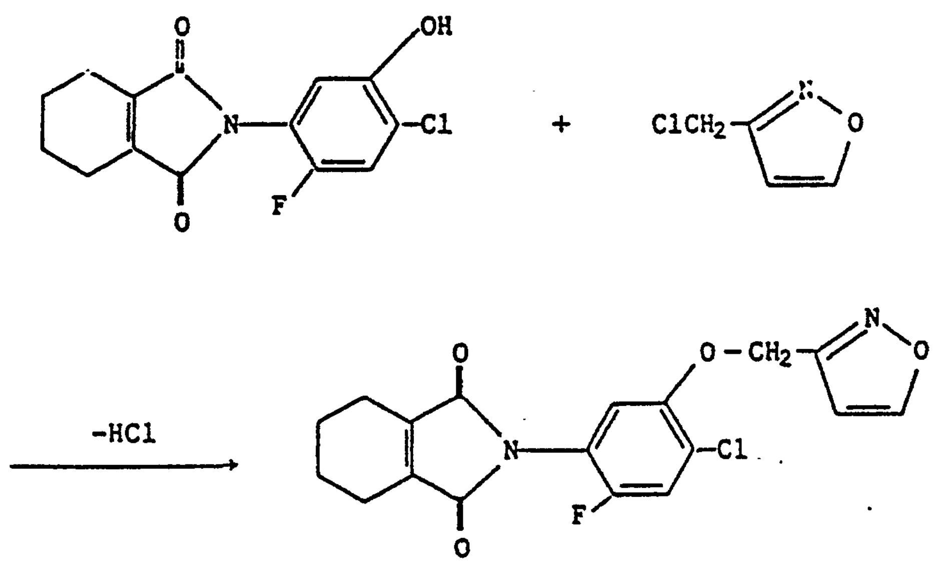

Wenn als Ausgangsstoff in dem Verfahren (c) beispielsweise (2-Ethoxycarbonyl-hexahydropyridazin-1-yl-carbonylamino)-4-chloro-2-fluoro-5-(pyridin-2-ylmethoxy)benzol eingesetzt wird, kann die Ringschluß-Kondensationsreaktion durch das folgende Schema dargestellt werden:

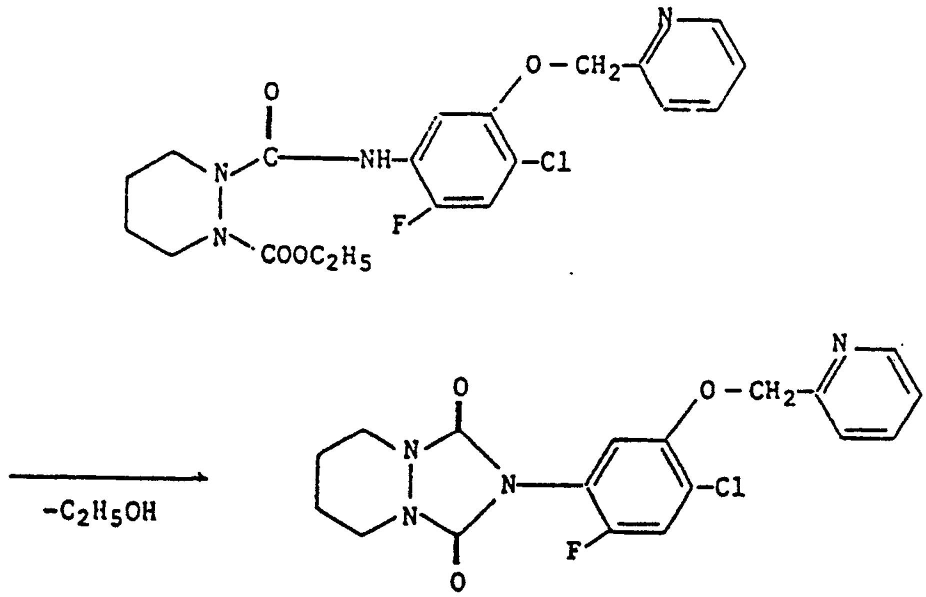


In dem Verfahren a) bezeichnen die Verbindungen der Formel (II) als Ausgangsstoffe solche auf der Grundlage der im Vorstehenden gegebenen Definitionen von $R^1$.

In der Formel (II) hat $R^1$ vorzugsweise die bereits im Vorstehenden genannten Bedeutungen.

Die Verbindungen der Formel (II) sind neu und können im allgemeinen mittels eines Verfahrens erhalten werden, bei dem

d) Verbindungen der Formel (V)

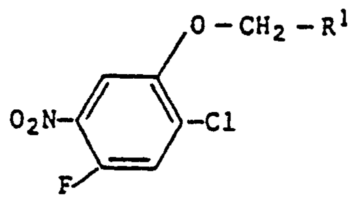

(V)

,

in der $R^1$ die im Vorstehenden angegebenen Bedeutungen hat,
in Gegenwart inerter Lösungsmittel reduziert werden.

Die Verbindungen der Formel (V) sind ebenfalls neu und können im allgemeinen mittels eines Verfahrens erhalten werden, bei dem

e) 2-Chloro-4-fluoro-5-nitrophenol mit dem im Vorstehenden bezeichneten Verbindungen der Formel (III) in Gegenwart inerter Lösungsmittel umgesetzt werden.

Die vorstehenden Verfahren d) und e) werden nach herkömmlichen Methoden durchgeführt.

Bei der Durchführung der Reduktionsreaktion bei dem Verfahren (d) ist es möglich, die Reaktion nach irgendeiner der verschiedenartigen Arbeitstechniken der Reduktion durchzuführen, beispielsweise einer katalytischen Reduktion mit Hilfe eines Katalysators wie Palladium, Raney-Nickel oder dergleichen, einer Reduktion mit Hilfe nascierenden Wasserstoffs, der durch Reaktion eines Metalls wie Eisen, Zink oder Zinn mit Wasser oder einer Säure erzeugt wurde, und einer Reduktion mit Hilfe einer Kombination aus Zinn(II)-chlorid und Salzsäure.

Das als Ausgangsstoff in dem Verfahren (b) verwendete 2-Chloro-4-fluoro-5-(4,5,6,7-tetrahydro-2H-isoindol-1,3-dion-2-yl)phenol kann in einfacher Weise aus 2-Chloro-4-fluoro-5-nitrophenol über 5-Amino-2-chloro-4-fluorophenol hergestellt werden, wie in den nachstehend angegebenen Beispielen erläutert ist.

In dem Verfahren b) bezeichnen die Verbindungen der Formel (III) als Ausgangsstoffe solche auf der Grundlage der im Vorstehenden gegebenen Definitionen von $R^1$ und M.

In der Formel (III) hat $R^1$ vorzugsweise die bereits im Vorstehenden genannten Bedeutungen, und M bezeichnet vorzugsweise Chlor oder Brom.

Die Verbindungen der Formel (III) sind auf dem Gebiet der organischen Chemie als Alkylierungsmittel wohlbekannt.

Das Chlor- oder Brom-Atom, das als der "abspaltende Rest" oder der "austretende Rest" in dem Alkylierungsmittel vorliegt, kann durch Iod, Methansulfonyloxy, Phenylsulfonyloxy, p-Toluolsulfonyloxy oder dergleichen ersetzt sein. Es ist auch möglich, ein Salz einer Halogenomethyl-heterocyclischen Verbindung und einer Mineralsäure, beispielsweise ein Hydrochlorid-Salz der genannten Verbindungen, einzusetzen.

Beispiele für die Verbindungen der Formel (III) sind:

2-(Chloromethyl)pyridin;
2-(Chloromethyl)pyrazin;
2-(Chloromethyl)pyrimidin;
4-(Chloromethyl)pyrimidin;
3-(Chloromethyl)pyridazin;
2-(Chloromethyl)furan;
3-(Chloromethyl)furan;
2-(Chloromethyl)thiophen;
3-(Chloromethyl)thiophen;
5-(Chloromethyl)-1-methyl-1H-1,2,4-triazol;
1-(Chloromethyl)-1H-1,2,4-triazol;
3-(Chloromethyl)isoxazol;
3-(Chloromethyl)-5-methylisoxazol;
5-(Chloromethyl)-3-methylisoxazol;
3-(Chloromethyl)-5-methyl-1,2,4-oxazol;
3-(Chloromethyl)-5-(methoxymethyl)-1,2,4-oxadiazol;
5-(Chloromethyl)-3-methyl-1,2,4-oxadiazol;
3-(Chloromethyl)-4-methyl-1,2,5-oxadiazol;
2-(Chloromethyl)-5-methyl-1,3,4-oxadiazol;
2-(Chloromethyl)thiazol;
4-(Chloromethyl)thiazol;
3-(Chloromethyl)-5-(methoxy)-1,2,4-thiadiazol;
3-(Chloromethyl)-5-(ethoxy)-1,2,4-thiadiazol;
3-(Bromomethyl)-1,2,5-thiadiazol;
2-(Chloromethyl)-5-methyl-1,3,4-thiadiazol etc..

In dem Verfahren c) bezeichnen die Verbindungen der Formel (IV) als Ausgangsstoffe solche auf der Grundlage der im Vorstehenden gegebenen Definitionen von $R^1$, $R^2$, $R^3$, Y und W. $R^5$ bezeichnet Wasserstoff oder $C_1$-$C_4$-Alkyl.

In der Formel (IV) haben $R^2$, $R^3$, Y, W und $R^1$ vorzugsweise die bereits im Vorstehenden genannten Bedeutungen, und $R^5$ bezeichnet vorzugsweise Wasserstoff oder Methyl.

Die Verbindungen der Formel (IV) sind ebenfalls neu und können im allgemeinen mittels eines Verfahrens erhalten werden, bei dem

f) Verbindungen der Formel (VI)

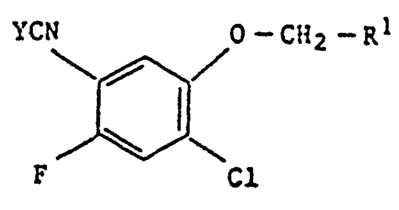

(VI)

in der Y und $R^1$ die im Vorstehenden angegebenen Bedeutungen haben, mit Verbindungen der Formel (VII)

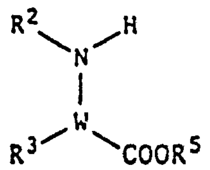

(VII)

in der W, $R^2$, $R^3$ und $R^5$ die im Vorstehenden angegebenen Bedeutungen haben,
in Gegenwart inerter Lösungsmittel umgesetzt werden.

Die Verbindungen der Formel (VI) sind ebenfalls neu und können im allgemeinen mittels eines Verfahrens erhalten werden, bei dem

g) die im Vorstehenden bezeichneten Verbindungen der Formel (II) mit Phosgen, Trichloromethylchlorameisensäureester oder Thiophosgen in Gegenwart inerter Lösungsmittel umgesetzt werden.

Die Isocyanate der Formel (VI) können mittels des Verfahrens g) nach einer in Org. Synthesis, Coll. Vol. 2, Seite 453, oder in J. Am. Chem. Soc. Vol. 72, Seite 1888, beschriebenen Methode erhalten werden, und die Isothiocyanate der Formel (VI) können mittels des Verfahrens g) nach einer in J. Org. Chem. 1953, Seite 1092, oder in J. Chem. Soc. 1927, Seite 1186, beschriebenen Methode erhalten werden.

Die Verbindungen der Formel (VII) in dem Verfahren f) sind bekannt.

Beispiele für die Verbindungen (VII) sind Ethylpipecolinat, 1-Ethoxycarbonyl-hexahydropyridazin und 1-Ethoxycarbonyl-1,2,3,6-tetrahydropyridazin.

Ethylpipecolinat, das in dem Verfahren f) eingesetzt wird, ist bekannt und im Handel erhältlich. 1-Ethoxycarbonyl-hexahydropyridazin kann mittels eines in Bull. Soc. Chim. France 1957, Seite 704, offenbarten Verfahrens oder eines in der US-PS 2 841 584 offenbarten Verfahrens hergestellt werden.

1-Ethoxycarbonyl-1,2,3,6-tetrahydropyridazin kann mittels eines in Bull. Soc. Chim. France 1957, Seite 704, offenbarten Verfahrens oder eines in der US-PS 2 841 584 offenbarten Verfahrens hergestellt werden.

Bei der Durchführung des Verfahrens f) ist es möglich, die angestrebte Reaktion nach einer in Agr. Biol. Chem. 40 [4], Seite 745, beschriebenen Methode herzustellen.

Die Verbindungen der Formeln (II), (V) und (VI) sind neu und können generisch durch die folgende Formel (VIII)

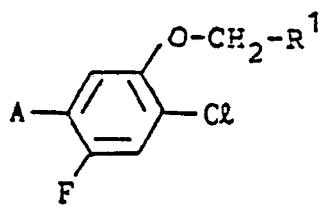

(VIII)

bezeichnet werden, in der
$R^1$ die im Vorstehenden angegebenen Bedeutungen hat und
A Amino, Nitro, Isocyanato oder Isothiocyanato bezeichnet.

Als geeignete Verdünnungsmittel für die Durchführung des Verfahrens a) kann jegliche Art organischer Lösungsmittel genannt werden. Bevorzugte Beispiele für die Verdünnungsmittel sind organische Säuren wie Essigsäure, Propionsäure etc..

Das Verfahren a) kann beispielsweise durch Erhitzen der Ausgangsstoffe in Essigsäure unter Rückfluß nach einer in Agr. Biol. Chem. 40, S. 745, beschriebenen Methode durchgeführt werden.

Es ist möglich, als geeignete Verdünnungsmittel für die Durchführung des Verfahrens b) beispielsweise Wasser, Nitrile wie Acetonitril, Alkohole wie Ethanol, Säureamide wie Dimethylformamid, Sulfoxide wie Dimethylsulfoxid, Ketone wie Aceton, Ether wie Tetrahydrofuran, Dioxan und Ethylenglycoldimethylether und dergleichen zu verwenden.

Das Verfahren b) kann in Gegenwart einer Base durchgeführt werden. Beispiele für solche Basen sind Natriumcarbonat, Natriumhydrid, Kaliumcarbonat, Kaliumhydroxid, Natriumhydroxid, Natriummethoxid, Natriumethoxid, Kalium-tert-butoxid und dergleichen.

Die Reaktionstemperatur des Verfahrens b) kann innerhalb eines Bereichs von beträchtlicher Breite variiert werden. Im allgemeinen wird die Reaktion bei einer Temperatur von etwa 10 °C bis 150 °C, vorzugsweise bei einer Temperatur von etwa 30 °C bis etwa 100 °C durchgeführt. Es wird bevorzugt, die

Reaktion unter normalem Druck durchzuführen, obwohl auch ein höherer oder niedrigerer Druck angewandt werden kann.

Bei der Durchführung des Verfahrens b) können beispielsweise 1 bis 1,2 mol der Verbindung der Formel (III) pro 1 mol 2-Chloro-4-fluoro-5-(4,5,6,7-tetrahydro-2H-isoindol-1,3-dion-2-yl)phenol eingesetzt werden. Diese Verbindungen werden miteinander in einem inerten Lösungsmittel in Gegenwart einer Base umgesetzt, so daß die gewünschten Verbindungen der Formel (I) erhalten werden können.

Wenn das Verfahren c) durchgeführt wird, ist es vorteilhaft, ein Verdünnungsmittel einzusetzen; zu solchen Verdünnungsmitteln zählen Alkohole wie Methanol und Ethanol, Ether wie Tetrahydrofuran, Dioxan und Dimethoxyethan, Benzol, Toluol, Xylol etc..

Das Verfahren c) kann bei einer Temperatur von etwa 20 °C bis 150 °C, vorzugsweise bei einer Temperatur von etwa 40 °C bis 100 °C durchgeführt werden.

Bei dem Verfahren c) ist es auch möglich, an Stelle von Natriummethoxid oder Natriumethoxid eine Base wie eine organische Base, darunter z.B. Triethylamin, Pyridin etc., und Natriumhydroxid, Kaliumhydroxid und dergleichen einzusetzen.

Das Verfahren c) kann nach einer in Agr. Biol. Chem. 40 [4], S. 745, beschriebenen Methode durchgeführt werden.

Die mittels des Verfahrens c) hergestellten gewünschten Verbindungen der Formel (I) können in einfacher Weise dadurch erhalten werden, daß die Verfahren f) und c) nacheinander durchgeführt werden, ohne die mittels des Verfahrens f) hergestellten Verbindungen der Formel (IV) zu isolieren.

Die erfindungsgemäßen Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die Verbindungen gemäß der Erfindung können beispielsweise in Verbindung mit den folgenden Pflanzen verwendet werden:

Dikotyledonen-Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen Verbindungen ist jedoch keineswegs auf diese Gattungen begrenzt, sondern umfaßt auch andere Pflanzen in gleicher Weise. In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Formulierungen wie Lösungen, Emulsionen, Suspensionen, Pulver, Schäume, Pasten, Granulat, Aerosole, mit dem Wirkstoff imprägnierte natürliche und synthetische Materialien, sehr feine Kapseln in polymeren Substanzen, Beschichtungsmittel zum Aufbringen auf Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Formulierungen können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit bekannten Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen 0,0005 und 3 kg/ha, vorzugsweise zwischen

0,001 und 2 kg/ha.

Die Herstellung und Verwendung der erfindungsgemäßen Verbindungen sind den folgenden Beispielen zu entnehmen.

Herstellungsbeispiele:

Beispiel 1

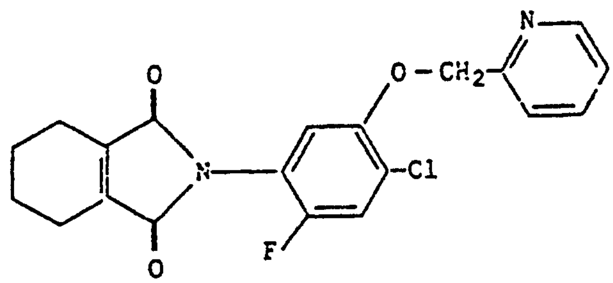

1,6 g 3,4,5,6-Tetrahydrophthalsäureanhydrid wurden zu einer Lösung von 2,53 g 4-Chloro-2-fluoro-5-(pyridin-2-ylmethoxy)anilin in 25 ml Essigsäure bei einer Temperatur von 10 °C bis 20 °C hinzugefügt. Die Reaktionsmischung wurde 1 h bei 20 °C bis 30 °C gerührt und dann 1,5 h zum Rückfluß erhitzt. Die Essigsäure wurde unter vermindertem Druck abdestilliert, und der resultierende Rückstand wurde in heißem Alkohol (80 bis 100 ml) gelöst und dann über Nacht auf 0 °C bis 5 °C gekühlt.

Das auf diese Weise gebildete kristalline Produkt wurde durch Filtration abgetrennt und an der Luft getrocknet, wonach 2,32 g der gewünschten Verbindung, d.h. 2-[4-Chloro-2-fluoro-5-(pyridin-2-ylmethoxy)-phenyl]-4,5,6,7-tetrahydro-2H-isoindol-1,3-dion erhalten wurden; Schmp. 124-127 °C.

Beispiel 2

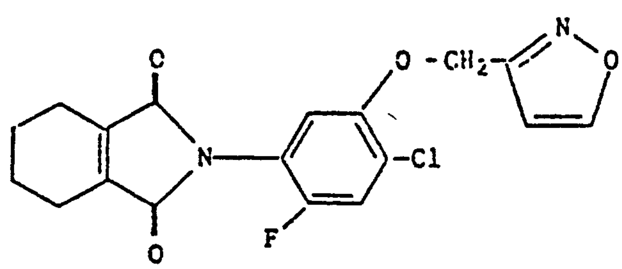

1,18 g 3-(Chloromethyl)isoxazol wurden tropfenweise bei einer Temperatur von 30 °C bis 40 °C zu einer Mischung von 2,96 g 2-Chloro-4-fluoro-5-(4,5,6,7-tetrahydro-2H-isoindol-1,3-dion-2-yl)phenol, 1,4 g Kaliumcarbonat und 30 ml Acetonitril hinzugefügt. Nach Beendigung der Zugabe wurde die Reaktionsmischung 3 h bei 50 °C bis 60 °C und dann 1 h unter Rückfluß und Rühren erhitzt um die Reaktion zu vervollständigen. Die Reaktionsmischung wurde auf Raumtemperatur abgekühlt und dann filtriert. Das Lösungsmittel wurde aus dem Filtrat abdestilliert, so daß ein sirupartiger Rückstand erhalten wurde. Der Rückstand wurde mit 80 ml Toluol und 20 ml Wasser vermischt, und die entstandene Mischung wurde gerührt und filtriert, um die Toluol-Schicht abzutrennen. Die Toluol-Schicht wurde mit einer 5-proz. wäßrigen Kaliumhydroxid-Lösung und dann mit Wasser gewaschen und über wasserfreiem Natriumsulfat getrocknet. Das Toluol wurde unter vermindertem Druck abdestilliert, und der erhaltene Rückstand wurde in 100 ml Ethanol unter Erhitzen gelöst. Die Ethanol-Lösung wurde mit Aktivkohle behandelt und dann bei 0 °C bis 5 °C über Nacht stehen gelassen. Das dabei gebildete kristalline Produkt wurde durch Filtration abgetrennt und an der Luft getrocknet, wonach 1,3 g der gewünschten Verbindung, d.h. 2-[4-Chloro-2-fluoro-5-(isoxazol-2-yl-methoxy)phenyl]-4,5,6,7-tetrahydro-2H-isoindol-1,3-dion erhalten wurden; Schmp. 155-157 °C.

Beispiel 3

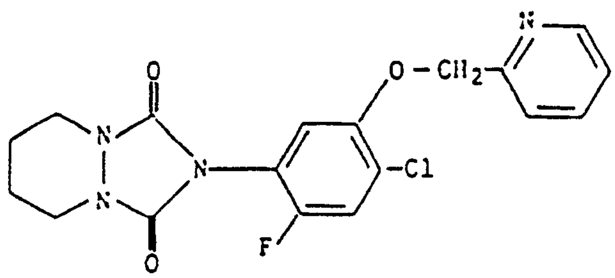

2,79 g 4-Chloro-2-fluoro-5-(pyridin-2-yl-methoxy)phenylisocyanat wurden in 30 ml Tetrahydrofuran gelöst. Zu der entstandenen Lösung wurden tropfenweise 1,58 g 1-Ethoxycarbonyl-hexahydropyridazin bei 10 °C bis 20 °C hinzugefügt. Die Reaktionsmischung wurde gerührt und dann über Nacht abkühlen gelassen. Das Tetrahydrofuran wurde unter vermindertem Druck abdestilliert. Der erhaltene Rückstand wurde mit 30 ml Ethanol und mit einer katalytischen Menge (0,06 g) Natriumethoxid vermischt. Die Reaktionsmischung wurde 5 h unter Rückfluß erhitzt. Das Ethanol wurde aus der Reaktionsmischung abdestilliert, und der erhaltene Rückstand wurde mit 100 ml Ethylacetat und mit 20 ml Wasser vermischt. Die resultierende Mischung wurde gerührt, und die organische Schicht wurde abgetrennt. Die organische Schicht wurde über wasserfreiem Natriumsulfat getrocknet und dann unter vermindertem Druck zur Trockne eingedampft. Das so erhaltene Produkt wurde aus einer Toluol/Hexan-Mischung umkristallisiert, wonach 3,0 g der gewünschten Verbindung, d.h. 4-[4-Chloro-2-fluoro-5-(pyridin-2-yl-methoxy)phenyl]-1,2-tetramethylen-1,2,4-triazolidin-3,5-dion erhalten wurden; Schmp. 162-163 °C.

Nach ähnlichen Verfahrensweisen wie in den oben angegebenen, in den Beispielen 1 bis 3 aufgezeigten, Verfahren können verschiedene Verbindungen der Formel (I) erhalten werden, wie in den Tabellen 1 und 2 gezeigt ist. Die Tabellen führen auch die in den Beispielen 1 bis 3 hergestellten Verbindungen auf.

Tabelle 1 zeigt Verbindungen der Formel (I), in denen Q den Rest

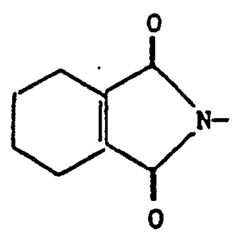

bezeichnet.

Tabelle 2 zeigt Verbindungen der Formel (I), in denen Q Q′ ist, das den Rest

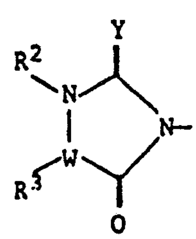

bezeichnet.

Tabelle 1

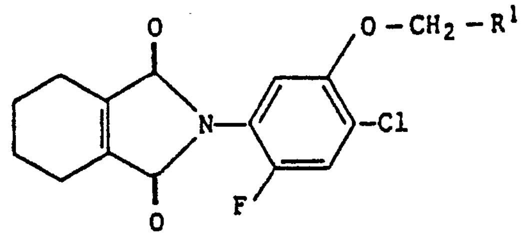

| Verbindung Nr. | $R^1$ | Physikalische Eigenschaften |
|---|---|---|
| 1 | Furan-2-yl | |
| 2 | Thiophen-2-yl | |
| 3 | 1-Methylpyrazol-3-yl | Viskose Flüssigkeit |
| 4 | 1-Methylimidazol-2-yl | Viskose Flüssigkeit |
| 5 | 1,2,4-Triazol-1-yl | |
| 6 | 1-Methyl-1,2,4-triazol-5-yl | Poröses Material |
| 7 | Isoxazol-3-yl | Schmp. 155-157 °C |
| 8 | 5-Methyl-isoxazol-3-yl | Schmp. 189-190 °C |
| 9 | 5-Methyl-1,2,4-oxadiazol-3-yl | Schmp. 126-130 °C |
| 10 | 3-Methyl-1,2,4-oxadiazol-5-yl | |
| 11 | 5-Ethyl-1,2,4-oxadiazol-3-yl | |
| 12 | 4-Methyl-1,2,5-oxadiazol-3-yl | Viskose Flüssigkeit |
| 13 | Thiazol-4-yl | Schmp. 117-119 °C |
| 14 | 5-Methoxy-1,2,4-thiadiazol-3-yl | Viskose Flüssigkeit |
| 15 | 5-Ethoxy-1,2,4-thiadiazol-3-yl | Viskose Flüssigkeit |
| 16 | 1,2,5-Thiadiazol-3-yl | Viskose Flüssigkeit |
| 17 | 4-Methyl-1,2,5-thiadiazol-3-yl | |
| 18 | Pyridin-2-yl | Schmp. 124-127 °C |
| 19 | Pyrazin-2-yl | Schmp. 152-155 °C |

## Tabelle 2

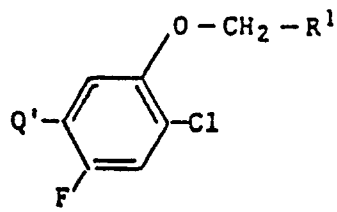

| Verbindung Nr. | Q' | $R^1$ | Physikalische Eigenschaften |
|---|---|---|---|
| 20 | 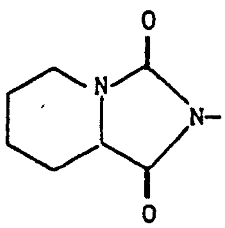 | Isoxazol-3-yl | |
| 21 | 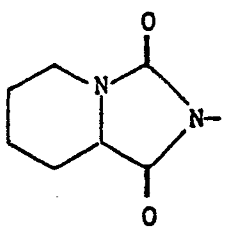 | Pyridin-2-yl | |
| 22 | 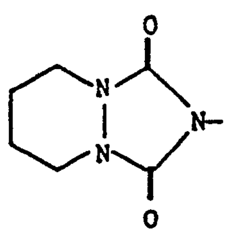 | 1,2,4-Triazol-1-yl | |
| 23 | 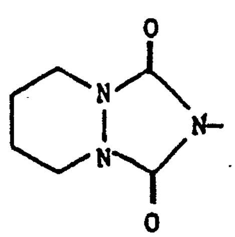 | Isoxazol-3-yl | |

Tabelle 2 - Fortsetzung

| Verbindung Nr. | Q' | $R^1$ | Physikalische Eigenschaften |
|---|---|---|---|
| 24 | 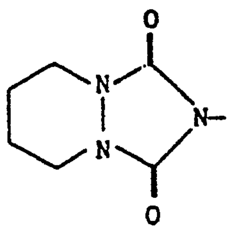 | 3-Methyl-1,2,4-oxadiazol-5-yl | |
| 25 | 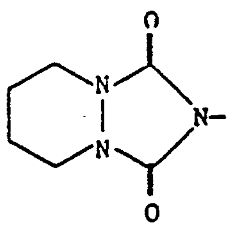 | 5-Methyl-1,2,4-oxadiazol-3-yl | |
| 26 | 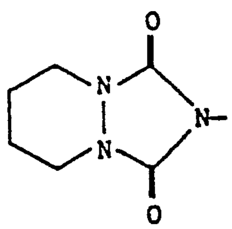 | 4-Methyl-1,2,5-oxadiazol-3-yl | |
| 27 | 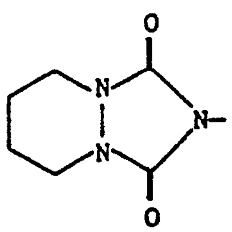 | Thiazol-4-yl | |
| 28 | 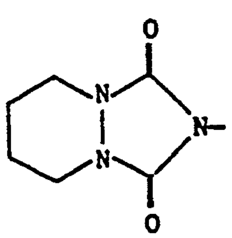 | Pyridin-2-yl | Schmp. 162-163 °C |

Tabelle 2 - Fortsetzung

| Verbindung Nr. | Q' | $R^1$ | Physikalische Eigenschaften |
|---|---|---|---|
| 29 | 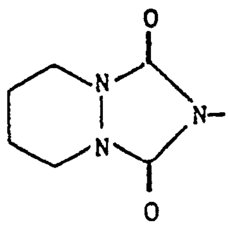 | Pyrazin-2-yl | |
| 30 | 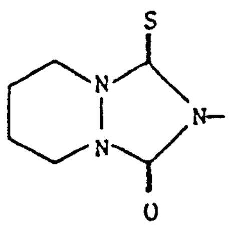 | Isoxazol-3-yl | Schmp. 136-138 °C |
| 31 | 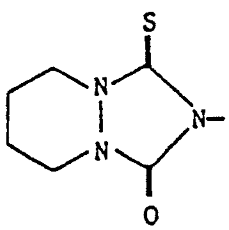 | 5-Methyl-1,2,4-oxadiazol-5-yl | |
| 32 | 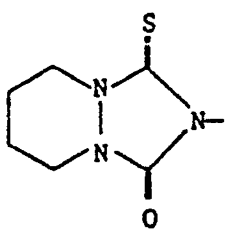 | Pyridin-2-yl | Schmp. 143-145 °C |
| 33 | 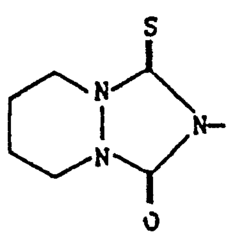 | Pyrazin-2-yl | |

Tabelle 2 - Fortsetzung

| Verbindung Nr. | Q' | $R^1$ | Physikalische Eigenschaften |
|---|---|---|---|
| 34 | 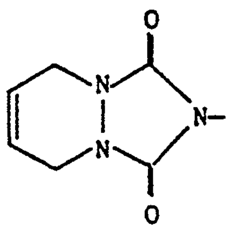 | Isoxazol-3-yl | |
| 35 | 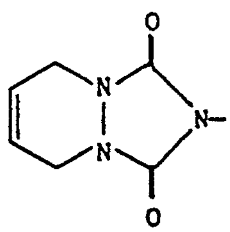 | Pyridin-2-yl | |
| 36 | 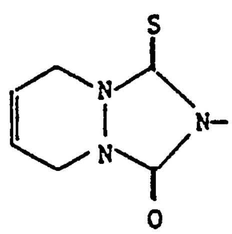 | Isoxazol-3-yl | |
| 37 | 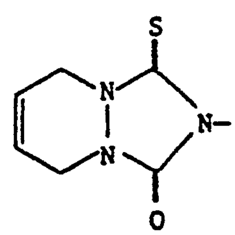 | Pyridin-2-yl | |

Beispiel 4

Herstellung eines Ausgangsstoffes (V-1)

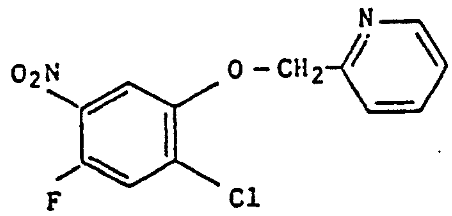

Eine Mischung aus 19,2 g 2-Chloro-4-fluoro-5-nitrophenol, 150 ml Acetonitril and 30,4 g Kaliumcarbonat wurde hergestellt. Zu dieser Mischung wurden portionsweise 18 g 2-(Chloromethyl)pyridin-hydrochlorid bei einer Temperatur von 20 °C bis 30 °C hinzugefügt. Nach dieser Zugabe wurde die Reaktionsmischung 5 h bei einer Temperatur von 50 °C bis 65 °C gerührt, und dann wurde die Reaktionsmischung gekühlt und filtriert. Das Filtrat wurde unter vermindertem Druck destilliert, um das Acetonitril daraus zu entfernen, und der erhaltene Rückstand wurde mit 200 ml Toluol und 100 ml Wasser vermischt. Die resultierende Mischung wurde gerührt, und dann wurde die dabei entstandene organische Schicht abgetrennt. Die organische Schicht wurde mit einer 5-proz. wäßrigen Kaliumhydroxid-Lösung und dann mit Wasser gewaschen, über wasserfreiem Natriumsulfat getrocknet und unter vermindertem Druck zur Trockne eingedampft. Das so erhaltene kristalline Rohprodukt wurde aus Ethanol umkristallisiert, wonach 21,7 g der gewünschten Verbindung, d.h. 4-Chloro-2-fluoro-5-(pyridin-2-yl-methoxy)nitrobenzol erhalten wurden; Schmp. 119-122 °C.

Beispiel 5

Herstellung eines Ausgangsstoffes (II-1)

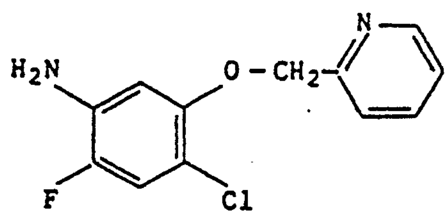

21,6 g der Verbindung 4-Chloro-2-fluoro-5-(pyridin-2-yl-methoxy)nitrobenzol, die in Beispiel 4 hergestellt worden war, wurden portionsweise zu einer Mischung von 26 g Eisen-Pulver, 160 ml Wasser, 7,8 g Essigsäure und 100 ml Ethanol bei einer Temperatur von 60 °C bis 70 °C unter Rühren hinzugefügt. Nach dieser Zugabe wurde die Reaktionsmischung 1 h bei einer Temperatur von 75 °C bis 85 °C gerührt. Nach Abdestillieren des Hauptteils des Ethanols unter Atmosphärendruck wurde die Reaktion auf Raumtemperatur abgekühlt. 300 ml Ethylacetat wurden zu der Reaktionsmischung hinzugefügt, und diese wurde danach genügend gerührt. Die Reaktionsmischung wurde durch eine Celit-Schicht (Filterhilfsmittel) filtriert. Die organische Schicht wurde von dem Filtrat abgetrennt und mit einer gesättigten wäßrigen Natriumhydrogencarbonat-Lösung und danach mit einer gesättigten Natriumchlorid-Lösung gewaschen und über wasserfreiem Natriumsulfat getrocknet. Das Lösungsmittel wurde abdestilliert, und der resultierende Rückstand wurde in Toluol gelöst, mit Aktivkohle behandelt und dann unter vermindertem Druck zur Trockne destilliert, wonach 10,7 g der gewünschten Verbindung, d.h. 4-Chloro-2-fluoro-5-(pyridin-2-yl-methoxy)anilin erhalten wurden; Schmp. 118-119 °C.

Beispiel 6

{Ausgangsstoff des Verfahrens b)}

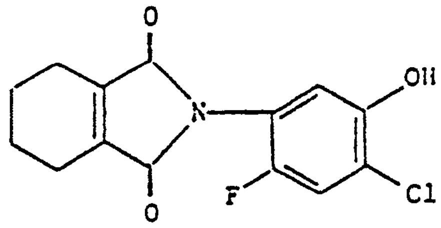

12 g 2-Chloro-4-fluoro-5-nitrophenol wurden mit 80 ml Essigsäure vermischt. Zu dieser Mischung wurden 2 g 10-proz. Pd/Kohlenstoff hinzugefügt. Danach wurde die Mischung der Reaktion mit Wasserstoff unter Atmosphärendruck unterworfen. Nach Aufnahme von 4,21 l Wasserstoff wurde die Reaktionsmischung durch eine Celit-Schicht filtriert. Der auf der Celit-Schicht abgelagerte Rückstand wurde mit Essigsäure (2mal 30 ml) gewaschen. Filtrat und Waschflüssigkeiten wurden vereinigt. Die erhaltene Lösung wurde mit 15,9 g 3,4,5,6-Tetrahydrophthalsäureanhydrid bei einer Temperatur von 20 °C bis 30 °C vermischt, 1 h bei 20 °C bis 30 °C gerührt und 3 h unter Rückfluß erhitzt. Die Essigsäure wurde unter vermindertem Druck abdestilliert. Der erhaltene Rückstand wurde in 250 ml Toluol gelöst, mit Wasser, einer gesättigten wäßrigen Natriumhydrogencarbonat-Lösung und wiederum mit Wasser gewaschen und über wasserfreiem Natriumsulfat getrocknet. Das Toluol wurde unter vermindertem Druck abdestilliert. Der Rückstand wurde aus einem Toluol/Hexan-Gemisch umkristallisiert, wonach 11,7 g der gewünschten Verbindung, d.h. 2-Chloro-4-fluoro-5-(4,5,6,7-tetrahydro-2H-isoindol-1,3-dion-2-yl-)phenol erhalten wurden; Schmp. 154-158 °C.

Biologische Tests

Zum Vergleich eingesetzte bekannte Verbindungen

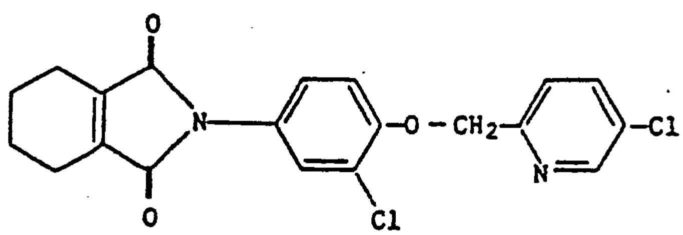

(E-1)

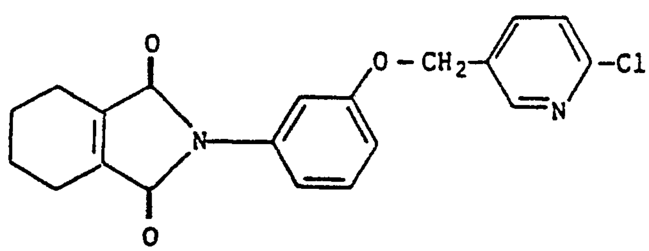

(E-2)

(Die Vergleichs-Verbindungen sind in der US-PS 4 332 944 beschrieben).

Beispiel 7

Test gegen Unkräuter in einem überfluteten Reisfeld durch Wasseroberflächen-Anwendung

Herstellung eines Wirkstoff-Präparats

Träger: 5 Gewichtsteile Aceton;
Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

Reis-Kulturboden wurde in Töpfe (5 $dm^2$; 25 cm x 20 cm x 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Schirmkraut (Cyperus difformis L.), Monochoria (Monochoria vaginalis) sowie einjährigen breitblättrigen Unkräutern [Falsche Pimpernelle (Lindernia pyxidaria L.), Rotala indica, Amerikanischer Wasserwurz (Elatine triandra), Rotstengel (Ammannia multiflora Roxburgh) und Dopatrium junceum Hamilton] wurden eingesät, und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats, mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |
| **Bewertung** | **Phytotoxizität gegen Nutzpflanze (bewertet unter Bezug auf die unbehandelte Fläche)** |
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind in Tabelle 3 aufgeführt.

Tabelle 3

| Verbindung Nr. | Wirkstoff-Menge kg/ha | Herbizide Wirkung gegen Unkräuter | | | | Phytotoxizität gegen Reis |
|---|---|---|---|---|---|---|
| | | Hühnerhirse | Schirmkraut | Monochoria | Breitblättrige Unkräuter | |
| 18 | 1,0 | 5 | 5 | 5 | 5 | 0 |
| | 0,5 | 4 | 5 | 5 | 5 | 0 |
| 19 | 1,0 | 5 | 5 | 5 | 5 | 1 |
| | 0,5 | 4 | 5 | 5 | 5 | 0 |
| Bekannte Verbindung | | | | | | |
| E-1 | 2,0 | 1 | 3 | 2 | 3 | 0 |
| | 1,0 | 0 | 2 | 1 | 1 | 0 |
| E-2 | 2,0 | 2 | 3 | 3 | 3 | 0 |
| | 1,0 | 1 | 2 | 2 | 2 | 0 |

Beispiel 8

Test gegen Unkräuter auf höher gelegenem Ackerboden Durch Bodenbehandlung vor dem Auflaufen:

In einem Gewächshaus wurden Sojabohnen-Samen in Töpfe von 500 $cm^2$ Fläche, die mit Ackererde gefüllt waren, eingesät, und Erde, die Samen von Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 7, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 7 geprüft. Die Ergebnisse sind in Tabelle 4 aufgeführt.

Tabelle 4

| Verbindung Nr. | Wirkstoff-Menge kg/ha | Herbizide Wirkung gegen Unkräuter | | | Phytotox. Effekt gegen Sojabohnen |
|---|---|---|---|---|---|
| | | Digitaria | Grauer Amaranth | Gänsefuß | |
| 6 | 0,5 | 5 | 5 | 5 | 1 |
| | 0,25 | 3 | 4 | 5 | 0 |
| 8 | 0,5 | 5 | 5 | 5 | 0 |
| | 0,25 | 3 | 5 | 5 | 0 |
| Bekannte Verbindung | | | | | |
| E-1 | 0,5 | 1 | 2 | 2 | 1 |
| E-2 | 0,5 | 1 | 2 | 2 | 0 |

Beispiel 9

Test gegen Unkräuter auf höher gelegenem Ackerboden durch Laub-Behandlung:

In einem Gewächshaus wurden Mais-Samen in Töpfe von 500 $cm^2$ Fläche, die mit Ackererde gefüllt waren, eingesät, und Erde, die Samen von Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 7, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 7 geprüft. Die Ergebnisse sind in Tabelle 5 aufgeführt.

Tabelle 5

| Verbindung Nr. | Wirkstoff-Menge kg/ha | Herbizide Wirkung gegen Unkräuter | | | Phytotoxizität gegen Mais-Pflanzen |
|---|---|---|---|---|---|
| | | Digitaria | Grauer Amaranth | Gänsefuß | |
| 6 | 0,5 | 5 | 5 | 5 | 0 |
| | 0,25 | 3 | 5 | 5 | 0 |
| 18 | 0,5 | 5 | 5 | 5 | 1 |
| | 0,25 | 4 | 5 | 5 | 0 |
| Bekannte Verbindung | | | | | |
| E-1 | 0,5 | 2 | 3 | 3 | 1 |
| E-2 | 0,5 | 2 | 2 | 3 | 0 |

**Ansprüche**

1. N-substituierte phenyl-heterocyclische Verbindungen der Formel (I)

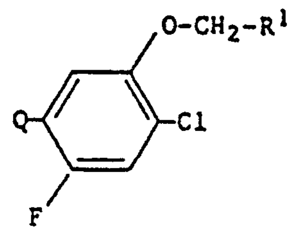

(I)

,

in der
Q die folgenden Gruppen

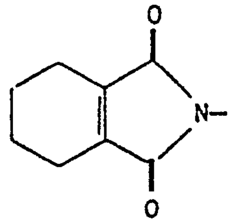

oder

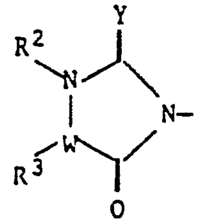

bezeichnet, worin
$R^2$ zusammen mit $R^3$ Tetramethylen oder die nachstehende Gruppe bildet

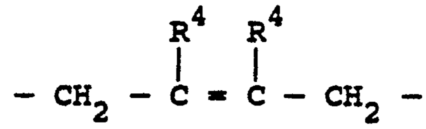 ,

$R^4$ Wasserstoff oder Methyl bezeichnet,
Y O oder S bezeichnet,
W - CH- oder - N - bezeichnet und
$R^1$ eine gegebenenfalls substituierte 5- oder 6-gliedrige heterocyclische Gruppe bezeichnet.

2. Verbindungen der Formel (I) nach Anspruch 1, dadurch gekennzeichnet, daß
Q die folgenden Gruppen

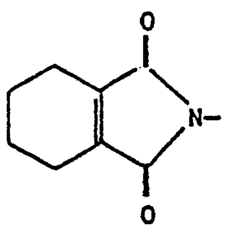 oder 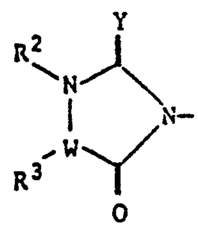

bezeichnet, worin
$R^2$ zusammen mit $R^3$ Tetramethylen oder die nachstehende Gruppe bildet
$-CH_2CH=CHCH_2-$ ,
Y O oder S bezeichnet,
W - CH- oder - N - bezeichnet und
$R^1$ eine gegebenenfalls substituierte 5-gliedrige heterocyclische oder 6-gliedrige aromatisch-heterocyclische Gruppe mit wenigstens einem Stickstoff-Atom bezeichnet.

3. Verbindungen der Formel (I) nach Anspruch 1, dadurch gekennzeichnet, daß
Q die folgenden Gruppen

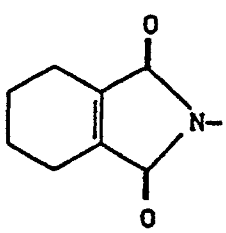 oder 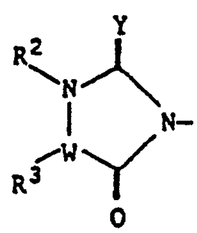

bezeichnet, worin
$R^2$ zusammen mit $R^3$ Tetramethylen oder die nachstehende Gruppe bildet
$-CH_2CH=CHCH_2-$ ,
Y O oder S bezeichnet,
W - CH- oder - N - bezeichnet und
$R^1$ eine gegebenenfalls substituierte 5-gliedrige heterocyclische Gruppe mit wenigstens einem Stickstoff-Atom und wenigstens einem Sauerstoff- oder Schwefel-Atom oder eine gegebenenfalls substituierte 6-gliedrige aromatisch-heterocyclische Gruppe mit 1 oder 2 Stickstoff-Atomen bezeichnet.

4. Verbindungen der Formel (I) nach Anspruch 1, nämlich
2-[4-Chloro-2-fluoro-5-(pyridin-2-yl-methoxy)phenyl]-4,5,6,7-tetrahydro-2H-isoindol-1,3-dion der Formel

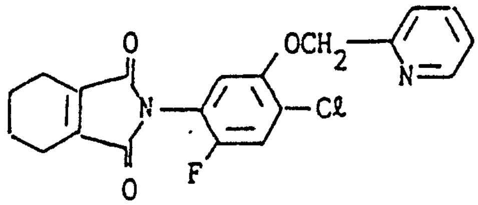

;

2-[4-Chloro-2-fluoro-5-(isoxazol-3-yl-methoxy)phenyl]-4,5,6,7-tetrahydro-2H-isoindol-1,3-dion der Formel

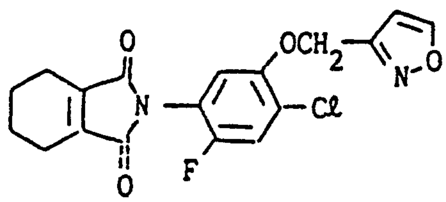

;

4-[4-Chloro-2-fluoro-5-(pyridin-2-yl-methoxy)phenyl]-1,2-tetramethylen-1,2,4-triazolidin-3,5-dion der Formel

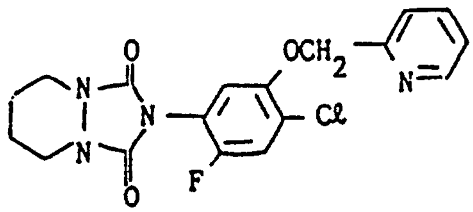

;

und
2-[4-Chloro-2-fluoro-5-(4-methyl-1,2,5-oxadiazol-3-yl-methoxy)phenyl]-4,5,6,7-tetrahydro-2H-isoindol-1,3-dion der Formel

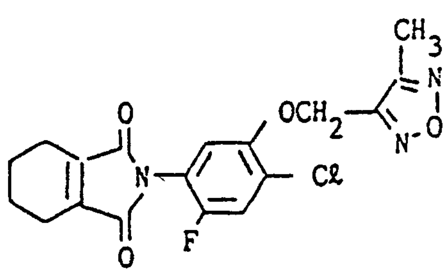

.

5. Verfahren zur Herstellung N-substituierter phenylheterocyclischer Verbindungen der Formel (I)

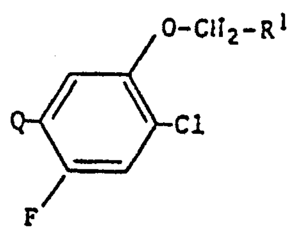

(I)

,

in der
Q die folgenden Gruppen

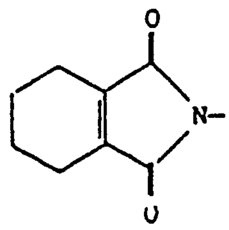 oder 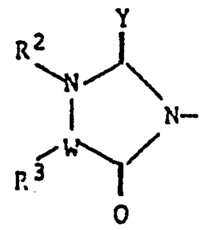

bezeichnet, worin
$R^2$ zusammen mit $R^3$ Tetramethylen oder die nachstehende Gruppe bildet

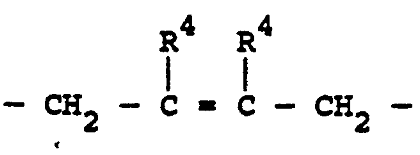 ,

$R^4$ Wasserstoff oder Methyl bezeichnet,
Y O oder S bezeichnet,
W - $\overset{|}{C}$H- oder - $\overset{|}{N}$ - bezeichnet und
$R^1$ eine gegebenenfalls substituierte 5- oder 6-gliedrige heterocyclische Gruppe bezeichnet,
dadurch gekennzeichnet, daß

a) in dem Fall, in dem Q die folgende Gruppe

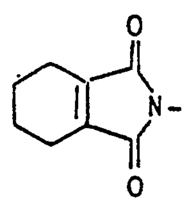

ist, Verbindungen der Formel (II)

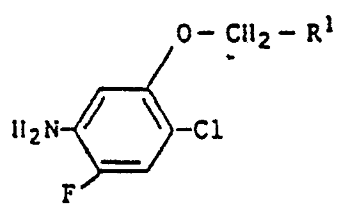 (II) ,

in denen $R^1$ die im Vorstehenden angegebenen Bedeutungen hat, mit 3,4,5,6-Tetrahydrophthalsäureanhydrid in Gegenwart inerter Lösungsmittel umgesetzt werden
oder

b) in dem Fall, in dem Q die folgende Gruppe

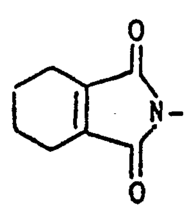

ist, 2-Chloro-4-fluoro-5-(4,5,6,7-tetrahydro-2H-isoindol-1,3-dion-2-yl)phenol mit Verbindungen der Formel

(III)

$M-CH_2-R^1$ (III),

in denen

$R^1$ die im Vorstehenden angegebenen Bedeutungen hat und

M Halogen, $C_1$-$C_4$-Alkylsulfonyloxy, Phenylsulfonyloxy oder p-Toluolsulfonyloxy bezeichnet, oder einem Salz der Verbindungen der Formel (III) in Gegenwart eines inerten Lösungsmittels und gegebenenfalls in Gegenwart einer Base umgesetzt werden

oder

c) in dem Fall, in dem Q die folgende Gruppe

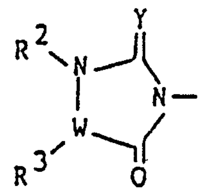

ist, Verbindungen der Formel (IV)

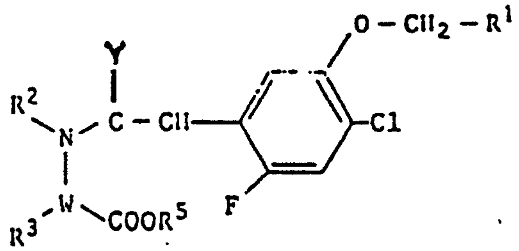

(IV)

in denen

$R^2$, $R^3$, Y, W und $R^1$ die im Vorstehenden angegebenen Bedeutungen haben und

$R^5$ Wasserstoff oder Niederalkyl bezeichnet, einer Ringschluß-Kondensationsreaktion in Gegenwart eines basischen Katalysators und in Gegenwart eines inerten Lösungsmittels unterworfen werden.

6. Herbizide Mittel, dadurch gekennzeichnet, daß sie wenigstens eine Verbindung der Formel (I) gemäß den Ansprüchen 1 bis 4 enthalten.

7. Verfahren zur Bekämpfung von Unkräutern, dadurch gekennzeichnet, daß Verbindungen der Formel (I) gemäß den Ansprüchen 1 bis 4 auf Unkräuter und/oder deren Lebensraum zur Einwirkung gebracht werden.

8. Verwendung von Verbindungen der Formel (I) gemäß den Ansprüchen 1 bis 4 zur Bekämpfung von Unkräutern.

9. Verfahren zur Herstellung herbizider Mittel, dadurch gekennzeichnet, daß Verbindungen der Formel (I) gemäß den Ansprüchen 1 bis 4 mit Streckmitteln und/oder grenzflächenaktiven Mitteln vermischt werden.

10. Verbindungen der Formel (VIII),

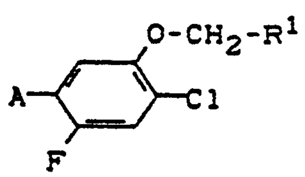

(VIII)

in denen

$R^1$ eine gegebenenfalls substituierte 5- oder 6-gliedrige heterocyclische Gruppe bezeichnet und

A Amino, Nitro, Isocyanato oder Isothiocyanato bezeichnet.

11. Verfahren zur Herstellung von Verbindungen der Formel (VIII),

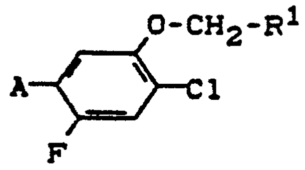

(VIII)

,

in denen
$R^1$ eine gegebenenfalls substituierte 5- oder 6-gliedrige heterocyclische Gruppe bezeichnet und
A Amino, Nitro, Isocyanato oder Isothiocyanato bezeichnet,
dadurch gekennzeichnet, daß

d) Verbindungen der Formel (V)

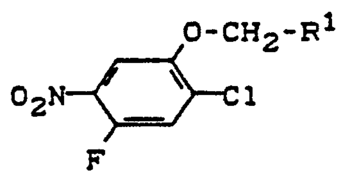

(V)

,

in der $R^1$ die im Vorstehenden angegebenen Bedeutungen hat,
in Gegenwart inerter Lösungsmittel reduziert werden
oder

e) 2-Chloro-4-fluoro-5-nitrophenol mit Verbindungen der Formel (III)

$M\text{-}CH_2\text{-}R^1$ (III),

in der
$R^1$ die im Vorstehenden angegebenen Bedeutungen hat und
M Halogen,$C_1$-$C_4$-Alkylsulfonyloxy, Phenylsulfonyloxy oder p-Toluolsulfonyloxy bezeichnet,
in Gegenwart inerter Lösungsmittel umgesetzt werden
oder

g) Verbindungen der Formel (II)

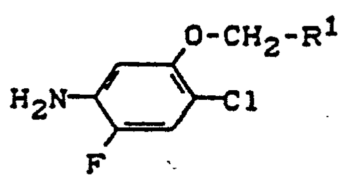

(II)

,

in der $R^1$ die im Vorstehenden angegebenen Bedeutungen hat, mit Phosgen, Trichloromethylchlorameisensäureester oder Thiophosgen in Gegenwart inerter Lösungsmittel umgesetzt werden.

12. Verbindungen der Formel (V)

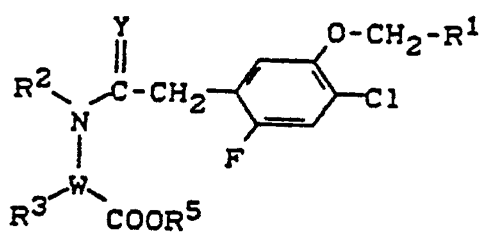

(V)

,

in der
$R^2$ zusammen mit $R^3$ Tetramethylen oder die nachstehende Gruppe bildet

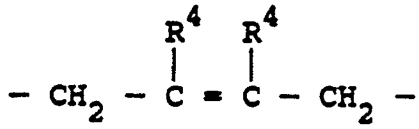

,

$R^4$ Wasserstoff oder Methyl bezeichnet,
Y O oder S bezeichnet,
W - CH- oder - N - bezeichnet,
$R^1$ eine gegebenenfalls substituierte 5- oder 6-gliedrige heterocyclische Gruppe bezeichnet und
$R^5$ Wasserstoff oder Niederalkyl bezeichnet.

13. Verfahren zur Herstellung von Verbindungen der Formel (V)

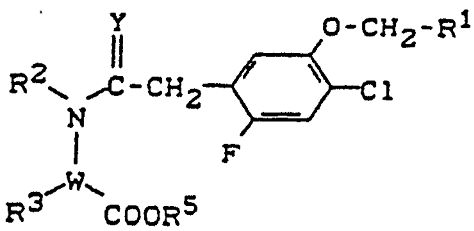

(V)

,

in der
$R^2$ zusammen mit $R^3$ Tetramethylen oder die nachstehende Gruppe bildet

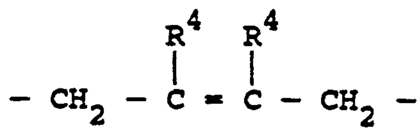

,

$R^4$ Wasserstoff oder Methyl bezeichnet,
Y O oder S bezeichnet,
W - CH- oder - N - bezeichnet,
$R^1$ eine gegebenenfalls substituierte 5- oder 6-gliedrige heterocyclische Gruppe bezeichnet und
$R^5$ Wasserstoff oder Niederalkyl bezeichnet,
dadurch gekennzeichnet, daß Verbindungen der Formel (VI)

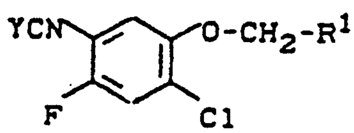

(VI)

,

in der Y und $R^1$ die im Vorstehenden angegebenen Bedeutungen haben, mit Verbindungen der Formel (VII)

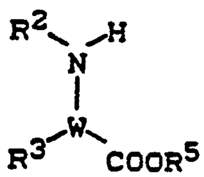

(VII)

,

in der W, $R^2$, $R^3$ und $R^5$ die im Vorstehenden angegebenen Bedeutungen haben, in Gegenwart inerter Lösungsmittel umgesetzt werden.